# EUROPEAN PATENT SPECIFICATION

(11) **EP 0 841 083 B1**
(45) Date of publication and mention of the grant of the patent: **28.07.2004**
(21) Application number: 97114876.2
(22) Date of filing: 27.08.1997
(51) Int. Cl.: B01D 35/143

(54) **Method and device for checking the condition of a filter in a ventilator**
Verfahren und Vorrichtung zur Prüfung von der Zustand eines Filters in einem Beatmungsgerät
Procédé et dispositif de vérification de l'état d'un filtre dans un respirateur

(30) Priority: 03.10.1996 SE 9603612
(43) Date of publication of application: 13.05.1998
(73) Proprietor: Maquet Critical Care AB, 171 95 Solna (SE)
(72) Inventor: Wallen, Lars, 163 60 Spanga (SE)
(74) Representative: Stein, Jan Anders Lennart

(56) References cited:
- DE-C- 2 404 452
- DE-C- 4 117 422
- GB-A- 2 253 572
- US-A- 5 477 731

## Description

The present invention relates to a method for proving at least one filter arranged in the flow paths of a breathing apparatus.

The present invention also relates to a device for proving at least one filter in the flow paths of a breathing equipment.

The present invention also relates to a breathing equipment according to the preamble to claim 9.

Filters of various kinds are frequently used in breathing equipment. In respiratory treatment, for example, bacterial filters are often employed to reduce the risk of a spread of infection between patients when the breathing equipment, usually a ventilator or respirator, is connected to a new patient. Bacterial filters can also be used to reduce the risk of exposing patients to bacteria carried in inspiratory air and the escape of bacteria in expiratory air into ambient air. Bacterial filters can also be used to protect equipment from "contaminants", such as medication and body fluids, in expiratory air.

The use of disposable filters, which are discarded after each use and replaced with new filters when a new patient is to be connected to the ventilator/respirator, is relatively common. Disposable filters represent a major cost to hospitals and also have an environmental impact. Bacterial filters are available which can be autoclaved and, accordingly, used for a plurality of patients, with inter-patient autoclaving to disinfect the filter. However, the disadvantage of reusing a filter is that it ultimately becomes clogged, thereby increasing resistance to flow in flow lines. A clogged filter can also increase pressure in the lung and increase the risk of injury. Filter leakage after the filter has been autoclaved a number of times is an additional risk. So these filters are discarded, while still fully serviceable, for safety reasons and are therefore not put to optimum use.

Disposable filters can naturally leak or become clogged as well, especially after a long period of use.

One object of the invention is to achieve a method for proving a filter in order to optimise filter life without any risk to patients. The check on condition especially applies to filters which can be used repeatedly with inter-patient cleaning/disinfection.

Another object of the invention is to achieve a device for proving filters in the flow paths of breathing equipment.

An additional object of the invention is to achieve breathing equipment with a built-in proving on filter condition.

One method for proving at least one filter arranged in the flow paths of the breathing apparatus is achieved according to the invention when the pressure drop across the filter is determined, the flow through the filter is determined, the pressure drop is related to the flow and the filter is deemed serviceable as long as the pressure drop remains within predefined limits in relation to the flow.

A filter always presents an impediment or resistance to the flow of a gas passing through the filter. This causes a drop in pressure which is related to both the filter's resistance and to flow. A clogged filter presents increased resistance, so the pressure drop for a given flow will increase across the clogged filter. Some clogging is acceptable, but the filter must be replaced when clogging become excessive. The magnitude of the pressure drop for every flow therefore becomes a measure of the condition of the filter.

Advantageous embodiments of the method are set forth in the dependent claims of claim 1.

A device for proving at least one filter in the flow paths of a breathing apparatus is achieved according to the invention when the device comprises a determination unit which is devised to prove the filter by using the method according to any of claims 1 - 4.

Advantageous embodiment of the device are set forth in the dependent claims of claim 5.

Breathing equipment is achieved according to the invention when a control unit in the breathing equipment comprises a device according to any of claims 5 - 8.

One advantageous embodiment of the breathing equipment is set forth in the dependent claim of claim 9.

Embodiments of the method, the device and the breathing equipment according to the invention will be described below in greater detail, referring to the figures in which
FIG. 1 shows one embodiment of breathing equipment according to the invention;
FIG. 2 shows one embodiment of a device according to the invention;
FIG. 3 shows a second embodiment of a device according to the invention.

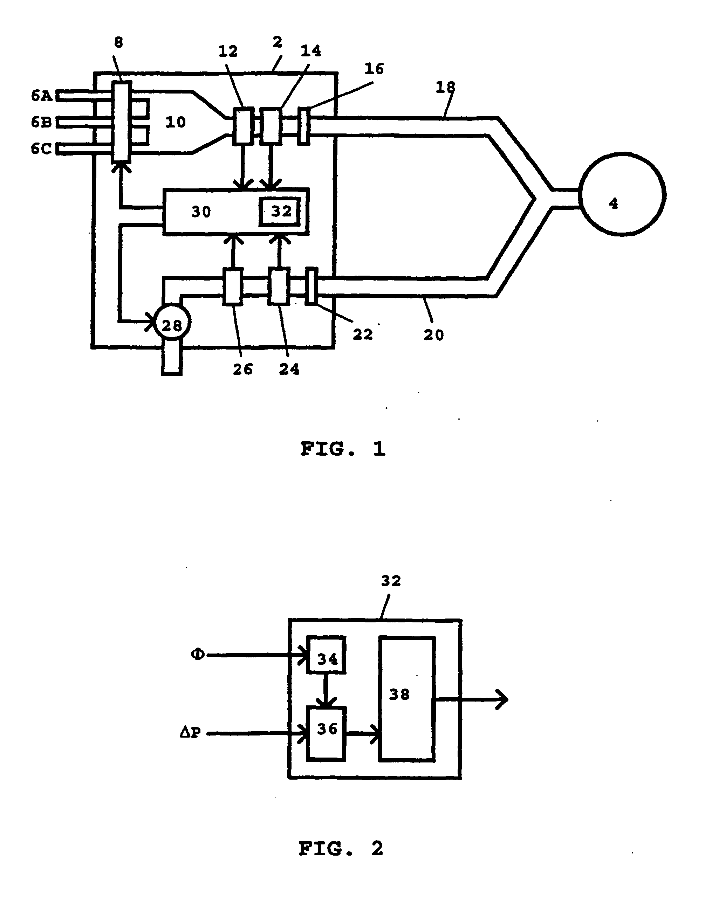

FIG. 1 shows a ventilator 2 connected to a patient 4 in order to supply her/him with a respiratory gas. The gas components jointly forming the respiratory gas are delivered to the ventilator 2 via a first gas connector 6A, a second gas connector 6B and a third gas connector 6C. The different gas components are regulated in a regulator unit 8 and then mixed in a mixing chamber 10. The mixed respiratory gas then passes a first flow meter 12, a first pressure meter 14 and a first bacterial filter 16, before being carried to the patient 4 via an inspiratory line 18. During exhalation, respiratory gas is carried from the patient 4 through an expiratory line 20 past a second bacterial filter 22, a second flow meter 24 and a second pressure meter 26. The respiratory gas then passes an expiratory valve 28 before being discharged into ambient air. All the functions in the ventilator 2 are controlled by a control device 30.

The first flow meter 12 can be replaced with flow measurements made by existing flow meters in the regulator unit 8 in order to regulate flows through the respective gas connector 6A, 6B, 6C. The sum of gas component flows then becomes the flow of the breathing gas. In the corresponding manner, the first pressure meter 14 can be replaced with existing pressure meters in the regulator unit 8.

A calculation unit 32 is arranged in the control device 30 for determining, from measured pressures and flows, the condition of the two bacterial filters 16, 22. During inhalation, when gas is only carried through the inspiratory line 18 to the patient 4, flow measurement values from the first flow meter 12 and pressures measured by the first pressure meter 14 and the second pressure meter 26 are used. The difference in pressure constitutes the drop in pressure across the first filter 16. The calculation unit 32 then relates the drop in pressure to the flow, and the first bacterial filter 16 is deemed to be fully serviceable if the drop in pressure lies within defined limits in relation to the flow.

If the drop in pressure is too great in relation to the flow, the first bacterial filter 16 is clogged and should therefore be replaced. If the drop in pressure in relation to flow suddenly falls during measurement over a plurality of consecutive inspirations, the first bacterial filter 16 is probably leaking and should therefore be replaced.

In the corresponding manner, the second bacterial filter 22 can be tested during expiration when gas only flows in the expiratory line 20. The flow is then determined by the second flow meter 24, and the drop in pressure is determined from the pressures measured by the first pressure meter 14 and the second pressure meter 26.

FIG. 2 shows one conceivable way in which the determination unit 32 could relate the drop in pressure to the flow. The measured flow ∅ is used as an input signal in a table 34. The table 34 holds information on the relationship between flow and pressure for a faultless bacterial filter, e.g. in the form of a measured flow-pressure curve for the specific filter type in question. The filter can be supplied with a bar code or the like which is read when the filter is installed. The correct curve is then automatically used by the determination unit 32. Certain safety limits, within which the filter is deemed to be serviceable, are also provided for the recorded curve. These safety margins can be established by tests of each filter type. The output signal from the table 34 then automatically becomes a pressure drop range which is sent to a comparator unit 36. The comparator unit 34 also uses as an input signal the pressure drop measured at the flow serving as the input signal for the table 34. The measured drop in pressure can then be compared in the comparator 36 to the upper limit and lower limit of the pressure drop range. As long as the pressure drop remains within the two limits, the filter can be regarded as serviceable. A signal to this effect is sent to a control unit 38 and can be shown on a display, turn on a green diode or lamp or be used in some way to indicate that the filter is serviceable. If the pressure drop exceeds the upper limit, the filter is clogged, and the comparator 36 sends a signal to this effect to the control unit 38. The control unit can, in turn, issue an alarm or inform the user, via a display or the like, that the filter should be replaced. In the corresponding manner, it can be determined that the filter should be replaced when the pressure drop falls below the lower limit.

Alternately, the comparator only issues a signal when the filter needs to be changed, irrespective of whether it is clogged or leaking. In digital respects, this can be when the comparator 36 has a zero at its output terminal as long as the filter is serviceable, i.e. the pressure drop lies within the limits. As soon as the pressure drop becomes greater or less, a one is generated, and an alarm indicating the need to replace the filter can be issued.

FIG. 3 shows a ventilator 40 connected to a patient 42. The ventilator 40 receives the gas components constituting the breathing gas via the gas connectors 44A, 44B, 44C, delivers breathing gas to the patient 42 via an inspiratory line 48 and evacuates gas from the patient 42 via an expiratory line 50.

In this instance, a first bacterial filter 52 is arranged in the inspiratory line 48, a first pressure meter 54 is arranged before the first filter 52 and a first flow meter 56 is arranged after the first filter 52. Measurement signals from the first flow meter 56 and the first pressure meter 54 are sent to a calculation unit 64 for a check on the condition of the filter 52. In the corresponding manner, a second filter 58 is arranged in the expiratory line 50 and a second pressure meter 60 and a second flow meter 62 are arranged in the expiratory line, whereupon the measurement signals are sent to the calculation unit 64. In the same way as noted above, the calculation unit 64 can determine the pressure drop across and flow through the respective filter during inspiration and expiration respectively and perform a proving of the filter. The calculation unit 64, the flow meters 56, 62 and the pressure meters 54, 60 constitute a device for checking the condition of the filters 52, 58.

The device can also receive measurement signals from existing pressure and flow meters in the ventilator 40. In principle, the device then only comprises the calculation unit 64, which is connected to the ventilator 40 to receive measurement signals for pressure and flow. In this embodiment, the filters can be located in the flow paths inside the ventilator 40.

A mixture of systems is also possible in which e.g. pressure signals are taken from the ventilator 40, and the device comprises flow meters 56, 62 and a calculation unit 64.

When only one filter is arranged in the flow paths, e.g. in the inspiratory part of the system, the condition of the filter can be checked in the corresponding manner by measuring pressure before and after the filter and measuring flow through the filter. The drop in pressure can be determined in the exact same way, i.e. from pressure signals from the inspiratory side and expiratory side respectively. Pressure meters can also be installed on either side of and in direct conjunction with the filter.

In order to reduce the risk of temporary disruptions, such as coughing, affects the proving, the relevant parameters can be studied over a plurality of breaths and processed statistically. This will also supply information for trend analysis in which remaining filter life can be calculated from changes occurring. Trend analysis can be of major value when there is a risk of rapid filter clogging, e.g. when medication or other "contaminants" are suspected of being or known to be present in expired air.

Combinations of the described embodiments are possible.

## Claims

1. A method for checking the condition of at least one filter arranged in an inspiration line or an expiration line of a medical ventilator between a first pressure meter and a first flow meter in the inspiratory line and a second pressure meter and a second flow meter in the expiratory line, the inspiratory line and expiratory line being in free flow communication with each other at one end and comprising a regulator unit for gas respectively an expiratory valve at respective other end, **characterized in that** the pressure drop across the filter is determined from measured pressures from the first and second pressure meters, flow through the filter is determined from the first flow meter or the second flow meter, the pressure drop is related to the flow and the filter is deemed to be serviceable as long as the pressure drop lies within predefined limits in relation to the flow.

2. A method according to claim 1, **characterized in that** the filter is deemed to be clogged if the pressure drop is greater than a predefined value in relation to the flow.

3. A method according to claim 1, **characterized in that** the relationship between pressure drop and flow is periodically determined during use, and the filter is deemed to be leaking if a change greater than a predefined threshold value occurs in the determined relationship.

4. Medical ventilator (2) comprising an inspiratory line (18), a regulator unit (8) for regulating a respiratory gas flow into the inspiratory line (18), a first pressure meter (14) for determining the pressure in the inspiratory line (18), a first flow meter (12) arranged to measure flow in the inspiratory line (18), an expiratory line (20) connected to the inspiratory line (18), an expiratory valve (28), a second pressure meter (26) for determining the pressure in the expiratory line (20), a second flow meter (24) arranged to measure flow in the expiratory line (20), at least one filter (16, 22) located in the inspiratory line (18) and/or expiratory line (20) and a control device (30) for controlling the ventilator (2), **characterized in that** the control device (30) comprises a determination unit (32), connected to the first and second pressure meters (14, 26) and to the first and second flow meters (12, 24) for receiving measurement signals from the first and second pressure meters (14, 26) and first and second flow meters (12, 24), the determination unit is adapted to determine the condition of the filter (16, 22) based on the relation between the pressure drop across the filter (16, 22) and flow through the filter (16, 22) whereby the filter (16, 22) is deemed to be serviceable as long as the pressure drop lies within predefined limits in relation to the flow.

5. Medical ventilator according to claim 4, **characterized in that** a first filter (16) is arranged in the inspiratory line (18), a second filter (22) is arranged in the expiratory line (20), and the determination unit (34) determines the pressure drop across the first filter (16) as the difference between pressures measured by the first pressure meter (14) and the second pressure meter (26) during inspiration and the pressure drop across the second filter (22) as the difference between pressures measured by the first pressure meter (14) and the second pressure meter (26) during expiration.

## Patentansprüche

1. Verfahren zur Prüfung des Zustandes wenigstens eines Filters, das in einer Inspirations- oder einer Exspirationsstrecke eines medizinischen Beatmungsgerätes zwischen einem ersten Druckmesser und einem ersten Durchflussmesser in der Inspirationsstrecke bzw. zwischen einem zweiten Druckmesser und einem zweiten Durchflussmesser in der Exspirationsstrecke angeordnet ist, wobei jeweils ein Ende der Inspirationsstrecke und ein Ende der Exspirationsstrecke miteinander in freier Durchflussverbindung stehen, während ihr jeweils anderes Ende mit einer Gas-Reguliereinheit bzw. mit einem Exspirationsventil versehen ist, und wobei das Verfahren **dadurch gekennzeichnet ist, dass** der Druckabfall am Filter aus dem vom ersten und zweiten Druckmesser jeweils gemessenen Druck ermittelt wird, während der Durchfluss durch das Filter vom ersten oder zweiten Durchflussmesser ermittelt wird, wobei sodann der Druckabfall in Beziehung zum Durchfluss gesetzt und das Filter so lange als einsatzfähig angesehen wird, als der Druckabfall in Beziehung zum Durchfluss innerhalb festgelegter Grenzwerte liegt.

2. Verfahren nach Anspruch 1, **dadurch gekennzeichnet, dass** das Filter als verstopft angesehen wird, wenn der Druckabfall in Beziehung zum Durchfluss einen festgelegten Wert übersteigt.

3. Verfahren nach Anspruch 1, **dadurch gekennzeichnet**, das die Beziehung zwischen Druckabfall und Durchfluss periodisch während des Einsatzes ermittelt wird und dass davon ausgegangen wird, dass das Filter leckt, wenn in der ermittelten Beziehung eine Veränderung eintritt, die einen festgelegten Schwellenwert übersteigt.

4. Medizinisches Beatmungsgerät (2), enthaltend eine Inspirationsstrecke (18), eine Reguliereinheit (8) zur Regulierung des Beatmungsgasflusses in der Inspirationsstrecke (18), einen ersten Druckmesser (14) zur Bestimmung des Drucks in der Inspirationsstrecke (18), einen ersten Durchflussmesser (12), der so angeordnet ist, dass er den Durchfluss in der Inspirationsstrecke (18) messen kann, eine Exspirationsstrecke (20), die mit der Inspirationsstrecke (18) verbunden ist, ein Exspirationsventil (28), einen zweiten Druckmesser (26) zur Bestimmung des Drucks in der Exspirationsstrecke (20), einen zweiten Durchflussmesser (24), der so angeordnet ist, dass er den Durchfluss in der Exspirationsstrecke (20) messen kann, wenigstens ein Filter (16, 22), das in der Inspirationsstrecke (18) und/oder in der Exspirationsstrecke (20) angeordnet ist, sowie eine zur Steuerung des Beatmungsgerätes (2) dienende Steuervorrichtung (30), wobei das Beatmungsgerät **dadurch gekennzeichnet ist, dass** die Steuervorrichtung (30) eine Bestimmungseinheit (32) umfasst, die an den ersten und zweiten Druckmesser (14, 26) und den ersten und zweiten Durchflussmesser (12, 24) so angeschlossen ist, dass sie vom ersten und zweiten Druckmesser (14, 26) und vom ersten und zweiten Durchflussmesser (12, 24) Messsignale empfängt, wobei die Bestimmungseinheit so ausgelegt ist, dass sie den Zustand des Filters (16, 22) auf der Grundlage der Beziehung zwischen dem Druckabfall am Filter (16, 22) und dem Durchfluss durch das Filter (16, 22) bestimmen kann, wobei das Filter (16, 22) so lange als einsatzfähig angesehen wird, als der Druckabfall in Beziehung zum Durchfluss innerhalb festgelegter Grenzwerte liegt.

5. Medizinisches Beatmungsgerät nach Anspruch 4, **dadurch gekennzeichnet**, das ein erstes Filter (16) in der Inspirationsstrecke (18) und ein zweites Filter (22) in der Exspirationsstrecke (20) angeordnet ist und die Bestimmungseinheit (34) den Druckabfall am ersten Filter (16) als Differenz zwischen den durch den ersten Druckmesser (14) und den zweiten Druckmesser (26) während der Inspiration gemessenen Drücken und den Druckabfall am zweiten Filter (22) als Differenz zwischen den durch den ersten Druckmesser (14) und den zweiten Druckmesser (26) während der Exspiration gemessenen Drücken bestimmt.

## Revendications

1. Procédé de vérification de l'état d'au moins un filtre monté dans une ligne d'inspiration ou dans une ligne d'expiration d'un ventilateur médical, entre un premier manomètre et un premier débitmètre dans la ligne d'inspiration et un second manomètre et un second débitmètre dans la ligne d'expiration, la ligne d'inspiration et la ligne d'expiration étant en communication de courant libre l'une avec l'autre à une extrémité et comprenant une unité de régulateur pour du gaz respectivement une vanne d'expiration à l'autre extrémité respective, **caractérisé en ce que** l'on détermine la perte de charge dans le filtre à partir des pressions mesurées par les premier et second manomètres, on détermine le débit dans le filtre à partir du premier débitmètre ou du second débitmètre, on met en relation la perte de charge avec le débit et l'on considère que le filtre est bon pour le service tant que la perte de charge se trouve dans des limites définies à l'avance en relation avec le débit.

2. Procédé suivant la revendication 1, **caractérisé en ce que** l'on considère que le filtre est engorgé si la perte de charge est supérieure à une valeur définie à l'avance en relation avec le débit.

3. Procédé suivant la revendication 1, **caractérisé en ce que** l'on détermine périodiquement la relation entre la perte de charge et le débit, et l'on considère que le filtre fuit s'il se produit une modification plus grande qu'une valeur de seuil définie à l'avance dans la relation déterminée.

4. Ventilateur (2) médical comprenant une ligne (18), d'inspiration, une unité (8) de régulateur destinée à réguler un courant de gaz respiratoire dans la ligne (18) d'inspiration, un premier manomètre (14) destiné à déterminé la pression dans la ligne (18) d'inspiration, un premier débitmètre (12) monté de manière à mesurer le débit dans la ligne (18) d'inspiration, une ligne (20) d'expiration reliée à la ligne (18) d'inspiration, une valve (28) d'expiration, un second manomètre (26) pour déterminer la pression dans la ligne (20) d'expiration, un second débitmètre (24) monté de manière à mesurer le débit dans la ligne (20) d'expiration, au moins un filtre (16, 22) placé dans la ligne (18) d'inspiration et/ou dans la ligne (20) d'expiration et un dispositif (30) de commande du ventilateur (2), **caractérisé en ce que** le dispositif (30) de commande comprend une unité (32) de détermination, reliée au premier et second manomètres (14, 26) et au premier et second débitmètres (12, 24) destinée à recevoir des signaux de mesure des premier et second manomètres (14, 26) et des premier et second débitmètres (12, 24), l'unité de détermination étant apte à déterminer l'état du filtre (16, 22) sur la base de la relation entre la perte de charge dans le filtre (16, 22) et le débit passant dans le filtre (16, 22) le filtre (16, 22) étant considéré comme bon pour le service tant que la perte de charge se trouve dans des limites définies à l'avance en relation avec le débit.

5. Ventilateur médical suivant la revendication 4, **caractérisé en ce qu'**un premier filtre (16) est monté dans la ligne (18) d'inspiration, un deuxième filtre (22) est monté dans la ligne (20) d'expiration, et l'unité (34) de détermination détermine la perte de charge dans le premier filtre (16) sous la forme de la différence entre des pressions mesurées par le premier manomètre (14) et le deuxième manomètre (26) pendant une inspiration et la perte de charge dans le deuxième filtre (22) sous la forme de la différence entre des pressions mesurées par le premier manomètre (14) et le deuxième manomètre (26) pendant une expiration.
